# FASCICULE DE BREVET EUROPEEN

(11) **EP 0 687 176 B1**
(45) Date de publication et mention de la délivrance du brevet: **30.05.2001**
(21) Numéro de dépôt: 94908374.5
(22) Date de dépôt: 25.02.1994
(51) Int. Cl.: A61K 31/425

(54) **APPLICATION DU RILUZOLE DANS LE TRAITEMENT DU NEURO-SIDA**
VERABREICHUNG VON RILUZOL ZUR BEHANDLUNG VON NEURO-AIDS
USE OF RILUZOLE FOR TREATING AIDS-RELATED NEURAL DISORDERS

(30) Priorité: 05.03.1993 FR 9302568
(43) Date de publication de la demande: 20.12.1995
(73) Titulaire: Aventis Pharma S.A., 92160 Antony (FR)
(72) Inventeur: BOUSSEAU, Anne, D-75011 Paris (FR); DOBLE, Adam, F-75005 Paris (FR); LOUVEL, Erik, F-75010 Paris (FR)
(74) Mandataire: Savina, Jacques
(86) Numéro de dépôt international: FR9400208
(87) Numéro de publication internationale: WO9420103

(56) Documents cités:
- EP-A- 0 050 551
- EP-A- 0 282 971
- EP-A- 0 558 861
- ANNUAL MEETING OF THE AMERICAN EPILEPSY SOCIETY, SEATTLE, WASHINGTON, USA, DECEMBER,, VOL. 33, SUPPL. 3, PAGE(S) 69, 1992 KOPPEL B et al 'ANTIEPILEPTIC DRUG TREATMENT IN PATIENTS WITH AIDS'
- DTSCH MED WOCHENSCHR (GERMANY, WEST), DEC 16 1988, VOL. 113, NO. 50, PAGE(S) 1975-81, Hartung HP et al 'Neuromuskulare Manifestationen der HIV-1- und HTLV-I-Infektionen.'
- J NEUROSCI (UNITED STATES), NOV 1989, VOL. 9, NO. 11, PAGE(S) 3720-7, Malgouris C et al 'Riluzole, a novel antiglutamate, prevents memory loss and hippocampal neuronal damage in ischemic gerbils.'
- CURR. OPIN. NEUROL. NEUROSURG. vol. 5, no. 4 , 1992 pages 508 - 513 B.S. MELDRUM ET AL. 'Excitatory amino acid receptors and disease'
- ARCH. NEUROL. vol. 48, no. 12 , 1991 pages 1281 - 1284 KIEBURTZ ET AL. 'Excitotoxicity and dopaminergic dysfunction in the Acquired Immunodeficiency Syndrome Dementia Complex'
- BR. J. PHARMACOL. vol. 97 , Juillet 1989 page 583P D. GIRDLESTONE ET AL. 'Riluzole antagonises excitatory amino acid-evoked firing in rat facial motoneurones in vivo'

## Description

La présente invention concerne une nouvelle application thérapeutique du riluzole ou les sels pharmaceutiquement acceptables de ce composé.

Le riluzole ou les sels pharmaceutiquement acceptables de ce composé sont décrits comme anticonvulsivants et antiépileptiques notamment dans le brevet EP 50551.

Il a maintenant été trouvé de manière surprenante que ce composé peut aussi être utilisé dans le traitement du neuro-SIDA.

Le terme neuro-SIDA comprend les troubles démentiels, les troubles cognitifs, les neuropathies, les myopathies, les troubles occulaires et tous les symptômes neurologiques liés au virus HIV-1.

L'activité du riluzole dans le neuro-SIDA a été mise en évidence dans le test de la mort neuronale induite par la protéine GP-120, protéine d'enveloppe du virus HIV-1 selon le protocole suivant :

Des cultures de cellules corticales sont préparées selon la méthode décrite par SINDOU et coll., Brain Res., 572, 242-246 (1992). Après 8 à 10 jours de culture, les neurones ayant acquis une forme neuritique correcte sont utilisés pour les tests. Les cellules sont gardées à 37°C dans une étuve à CO₂ pour l'ensemble de l'expérience.

La survie neuronale est appréciée avant et après 24 heures d'application du produit à tester par une technique colorimétrique au Bleu de Tuspan en comptant des champs prédéterminés (méthode semi-quantitative). Un minimum de 4 boîtes de cultures par concentration (100 neurones par boites) a été analysé.

Dans une première série, la survie neuronale du milieu de culture a été déterminée sans aucun produit. La survie neuronale est alors d'environ 87%.

Dans une deuxième série, la toxicité de la GP120 en culture a été mise en évidence. La GP120 a été appliquée sur le milieu de culture pendant 24 heures seule à une concentration de 20 pmol et entraîne une mort neuronale de l'ordre de 43%.

Dans la troisième série, le produit à tester en solution dans le diméthylsulfoxyde (10⁻³ M) est appliqué 5 minutes avant l'application de GP120 et incubé ensuite pendant 24 heures à des concentrations de 10⁻⁷ à 10⁻⁸ mol.. Les résultats obtenus sont les suivants :

| SERIE 1 TEMOINS SURVIE NEURONALE | SERIE 2 SURVIE NEURONALE GP120 seul (20pmol) | SERIE 3 SURVIE NEURONALE GP120 et riluzole (10⁻⁷mol) |
|---|---|---|
| 87,2 ± 7,2% | 57,3 ± 9,1% | 80,6 ± 9,9% |

Comme sels pharmaceutiquement acceptables peuvent être notamment cités les sels d'addition avec les acides minéraux tels que chlorhydrate, sulfate, nitrate, phosphate ou organiques tels que acétate, propionate, succinate, oxalate, benzoate, fumarate, maléate, méthanesulfonate, iséthionate, théophilline-acétate, salicylate, phénolphtalinate, méthylène-bis-β-oxynaphtoate ou des dérivés de substitution de ces dérivés.

Les médicaments sont constitués par au moins le riluzole sous forme libre ou sous forme d'un sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale ou parentérale.

Comme compositions solides pour administration orale, peuvent être utilisés des comprimés des pilules des poudres (cansules de gélatine cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les doses dépendent de l'effet recherché, de la durée du traitement et de la voie d'administration utilisée ; elles sont généralement comprises entre 50 et 400 mg par jour par voie orale pour un adulte avec des doses unitaires allant de 25 à 200 mg de substance active.

D'une façon générale, le médecin déterminera la posologie appropriée en fonction de l'âge, du poids et de tous les autres facteurs propres au sujet à traiter.

Les exemples suivants illustrent des médicaments selon l'invention :

### Exemple A

On prépare, selon la technique habituelle, des comprimés dosés à 50 mg de produit actif ayant la composition suivante :
- Riluzole 50 mg
- Mannitol 64 mg
- Cellulose microcristalline 50 mg
- Polyvidone excipient 12 mg
- Carboxyméthylamidon sodique 16 mg
- Talc 4 mg
- Stéarate de magnésium 2 mg
- Silice colloïdale anhydre 2 mg
- Mélange de méthylhydroxypropylcellulose,
   polyéthylèneglycol 6000, dioxyde de titane (72-3,5-24,5)
   q.s.p. 1 comprimé pelliculé terminé à 245 mg

### Exemple B

On prépare, selon la technique habituelle, des gélules dosées à 50 mg de produit actif ayant la composition suivante :
- Riluzole 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### Exemple C

On prépare une solution injectable contenant 10 mg de produit actif ayant la composition suivante :
- Riluzole 10 mg
- Acide benzoïque 80 mg
- Alcool benzylique 0,06 cm3
- Benzoate de sodium 80 mg
- Ethanol à 95 % 0,4 cm3
- Hydroxyde de sodium 24 mg
- Propylène glycol 1,6 cm3
- Eau q.s.p 4 cm3

L'invention concerne également le procédé de préparation de médicaments utiles dans le traitement du neuro-SIDA consistant à mélanger le riluzole ou les sels pharmaceutiquement acceptables de ce composé avec un ou plusieurs diluants et/ou adjuvants compatibles et pharmaceutiquement acceptables.

## Revendications

1. Application du riluzole ou les sels pharmaceutiquement acceptables de ce composé à la préparation de médicaments destinés au traitement du neuro-SIDA.

2. Application selon la revendication 1 pour la préparation de médicaments destinés au traitement des troubles démentiels, des troubles cognitifs, des neuropathies, de la myopathie, des troubles occulaires et de tous les symptômes neurologiques liés au virus HIV 1.

3. Application selon la revendication 1 pour obtenir un médicament comprenant 25 à 200 mg du riluzole.

4. Application du riluzole ou les sels pharmaceutiquement acceptables de ce composé à la préparation de médicaments destinés à diminuer la mort neuronale induite par la proteine GP-120" .

## Patentansprüche

1. Verwendung von Riluzol oder den pharmazeutisch verträglichen Salzen dieser Verbindung zur Herstellung von Medikamenten zur Behandlung von Neuro-AIDS.

2. Verwendung nach Anspruch 1 zur Herstellung von Medikamenten zur Behandlung von Demenz-Störungen, kognitiven Störungen, Neuropathien, Myopathie, Störungen der Augen und allen neurologischen Symptomen im Zusammenhang mit dem HIV 1-Virus.

3. Verwendung nach Anspruch 1 zum Erhalt eines Medikaments, das 25 bis 200 mg Riluzol umfasst.

4. Verwendung von Riluzol oder den pharmazeutisch verträglichen Salzen dieser Verbindung zur Herstellung von Medikamenten zur Verminderung des neuronalen Tods, der durch das Protein GP-120 induziert wird.

## Claims

1. Use of riluzole or the pharmaceutically acceptable salts of this compound for the preparation of medicinal products intended for the treatment of neuro-AIDS.

2. Use according to Claim 1, for the preparation of medicinal products intended for the treatment of disorders involving dementia, cognitive disorders, neuropathies, myopathy, ocular disorders and all neurological symptoms associated with the HIV-1 virus.

3. Use according to Claim 1, for obtaining a medicinal product comprising 25 to 200 mg of riluzole.

4. Use of riluzole or the pharmaceutically acceptable salts of this compound for the preparation of medicinal products intended for decreasing neuronal death induced by the GP-120" protein.
